(19) Europäisches Patentamt
European Patent Office
Office européen des brevets

(11) EP 2 239 325 A1

(12) **EUROPEAN PATENT APPLICATION**
published in accordance with Art. 153(4) EPC

(43) Date of publication:
**13.10.2010 Bulletin 2010/41**

(21) Application number: **09702346.9**

(22) Date of filing: **13.01.2009**

(51) Int Cl.:
***C12N 15/09*** (2006.01) ***A61K 35/76*** (2006.01)
***A61P 9/10*** (2006.01) ***A61P 11/00*** (2006.01)
***A61P 25/00*** (2006.01) ***A61P 35/00*** (2006.01)
***C07K 14/03*** (2006.01) ***C07K 14/47*** (2006.01)
***C07K 19/00*** (2006.01) ***C12N 7/00*** (2006.01)

(86) International application number:
**PCT/JP2009/050299**

(87) International publication number:
**WO 2009/090933 (23.07.2009 Gazette 2009/30)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **16.01.2008 JP 2008007179**

(71) Applicants:
- **Japan Science and Technology Agency**
  **Kawaguchi-shi**
  **Saitama 332-0012 (JP)**
- **Takahashi, Katsuhito**
  **Kyoto 602-0827 (JP)**
- **Yamamura, Hisako**
  **Ikoma-shi**
  **Nara 630-0247 (JP)**

(72) Inventors:
- **TAKAHASHI, Katsuhito**
  **Kyoto-shi**
  **Kyoto 602-0827 (JP)**
- **YAMAMURA, Hisako**
  **Ikoma-shi**
  **Nara 630-0247 (JP)**
- **INOUE, Masahiro**
  **Suita-shi**
  **Osaka 565-0872 (JP)**

(74) Representative: **Baldock, Sharon Claire**
**Boult Wade Tennant**
**Verulam Gardens**
**70 Gray's Inn Road**
**London WC1X 8BT (GB)**

(54) **VIRUS GROWING IN HYPOXIC CELL OR VIRUS VECTOR EXPRESSING GENE THEREIN**

(57) The present invention provides a virus or a viral vector capable of expressing a gene specifically in a cell having replication ability in a hypoxic state such as a cancer stem cell and injuring the cell, and a pharmaceutical composition comprising the same. Specifically, the present invention provides a virus or a viral vector which comprises a gene encoding a fusion protein of an ODD and a protein essentially required for viral proliferation, and a pharmaceutical composition comprising the same.

FIG. 3

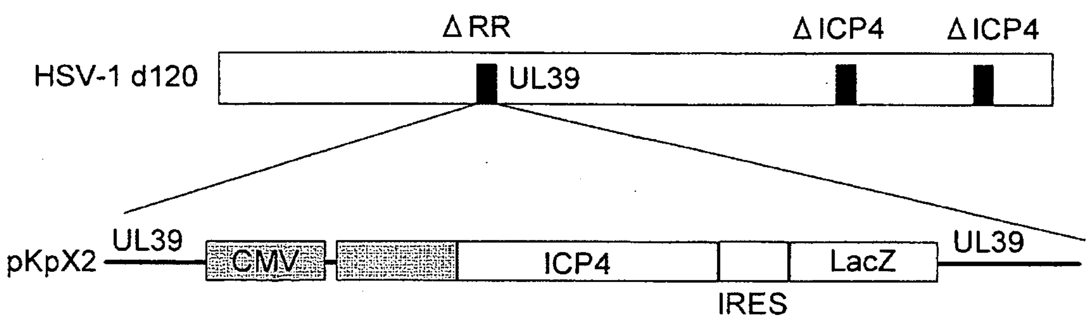


EP 2 239 325 A1

SEQUENCE LISTING

<110> Japan Science and Technology Agency
TAKAHASHI, Katsuhito
YAMAMURA, Hisako

<120> Virus proliferating under hypoxic condition or virus vector for expression of gene

<130> P113216EP00

<140> EP09702346.9
<141> 2009-01-13

<150> PCT/JP2009/050299
<151> 2009-01-13

<150> JP 2008-007179
<151> 2008-01-16

<160> 6

<170> PatentIn version 3.2

<210> 1
<211> 56
<212> PRT
<213> Artificial Sequence

<220>
<223> ODD

<400> 1
```
Asn Pro Phe Ser Thr Gln Asp Thr Asp Leu Asp Leu Glu Met Leu Ala
1               5                   10                  15

Pro Tyr Ile Asp Met Asp Asp Phe Gln Leu Arg Ser Phe Asp Gln Leu
            20                  25                  30

Ser Pro Leu Glu Ser Ser Ser Ala Ser Pro Glu Ser Ala Ser Pro Gln
        35                  40                  45

Ser Thr Val Thr Val Phe Gln Gln
    50                  55
```

<210> 2
<211> 171
<212> DNA
<213> Artificial Sequence

<220>
<223> ODD

<400> 2
```
aacccatttt ctactcagga cacagattta gacttggaga tgttagctcc ctatatccca      60
atggatgatg acttccagtt acgttccttc gatcagttgt caccattaga aagcagttcc     120
gcaagccctg aaagcgcaag tcctcaaagc acagttacag tattccagca g              171
```

<210> 3
<211> 79
<212> PRT

```
<213>  Artificial Sequence

<220>
<223>  NLS-ODD

<400>  3
Met Ala Pro Lys Lys Lys Arg Lys Arg Ser Tyr Gly Arg Lys Lys Arg
1               5                   10                  15

Arg Gln Arg Arg Arg Arg Ser Asn Pro Phe Ser Thr Gln Asp Thr Asp
            20                  25                  30

Leu Asp Leu Glu Met Leu Ala Pro Tyr Ile Asp Met Asp Asp Phe Gln
        35                  40                  45

Leu Arg Ser Phe Asp Gln Leu Ser Pro Leu Glu Ser Ser Ser Ala Ser
    50                  55                  60

Pro Glu Ser Ala Ser Pro Gln Ser Thr Val Thr Val Phe Gln Gln
65                  70                  75

<210>  4
<211>  255
<212>  DNA
<213>  Artificial Sequence

<220>
<223>  Kozak-NLS-ODD

<400>  4
aattcccagc ttgacatggc gcctaagaag aagaggaaga gatcatatgg tcgtaagaaa      60
cgtcgccaac gtcgccgaag atctaaccca ttttctactc aggacacaga tttagacttg     120
gagatgttag ctccctatat cccaatggat gatgacttcc agttacgttc cttcgatcag     180
ttgtcaccat tagaaagcag ttccgcaagc cctgaaagcg caagtcctca aagcacagtt     240
acagtattcc agcag                                                       255

<210>  5
<211>  1379
<212>  PRT
<213>  Artificial Sequence

<220>
<223>  ODD-ICP4 fusion protein

<400>  5
Met Ala Pro Lys Lys Lys Arg Lys Arg Ser Tyr Gly Arg Lys Lys Arg
1               5                   10                  15

Arg Gln Arg Arg Arg Arg Ser Asn Pro Phe Ser Thr Gln Asp Thr Asp
            20                  25                  30

Leu Asp Leu Glu Met Leu Ala Pro Tyr Ile Asp Met Asp Asp Phe Gln
        35                  40                  45

Leu Arg Ser Phe Asp Gln Leu Ser Pro Leu Glu Ser Ser Ser Ala Ser
    50                  55                  60

Pro Glu Ser Ala Ser Pro Gln Ser Thr Val Thr Val Phe Gln Gln Val
65                  70                  75                  80

Pro Met Ala Ser Glu Asn Lys Gln Arg Pro Gly Ser Pro Gly Pro Thr
```

```
                85                  90                  95
Asp Gly Pro Pro Pro Thr Pro Ser Pro Asp Arg Asp Glu Arg Gly Ala
            100                 105                 110
Leu Gly Trp Gly Ala Glu Thr Glu Glu Gly Gly Asp Asp Pro Asp His
        115                 120                 125
Asp Pro Asp His Pro His Asp Leu Asp Asp Ala Arg Arg Asp Gly Arg
    130                 135                 140
Ala Pro Ala Ala Gly Thr Asp Ala Gly Glu Asp Ala Gly Asp Ala Val
145                 150                 155                 160
Ser Pro Arg Gln Leu Ala Leu Leu Ala Ser Met Val Glu Glu Ala Val
                165                 170                 175
Arg Thr Ile Pro Thr Pro Asp Pro Ala Ala Ser Pro Pro Arg Thr Pro
            180                 185                 190
Ala Phe Arg Ala Asp Asp Asp Asp Gly Asp Glu Tyr Asp Asp Ala Ala
        195                 200                 205
Asp Ala Ala Gly Asp Arg Ala Pro Ala Arg Gly Arg Glu Arg Glu Ala
    210                 215                 220
Pro Leu Arg Gly Ala Tyr Pro Asp Pro Thr Asp Arg Leu Ser Pro Arg
225                 230                 235                 240
Pro Pro Ala Gln Pro Pro Arg Arg Arg Arg His Gly Arg Trp Arg Pro
                245                 250                 255
Ser Ala Ser Ser Thr Ser Ser Asp Ser Gly Ser Ser Ser Ser Ser Ser
            260                 265                 270
Ala Ser Ser Ser Ser Ser Ser Ser Asp Glu Asp Glu Asp Asp Asp Gly
        275                 280                 285
Asn Asp Ala Ala Asp His Ala Arg Glu Ala Arg Ala Val Gly Arg Gly
    290                 295                 300
Pro Ser Ser Ala Ala Pro Ala Ala Pro Gly Arg Thr Pro Pro Pro Pro
305                 310                 315                 320
Gly Pro Pro Pro Leu Ser Glu Ala Ala Pro Lys Pro Arg Ala Ala Ala
                325                 330                 335
Arg Thr Pro Ala Ala Ser Ala Gly Arg Ile Glu Arg Arg Arg Ala Arg
            340                 345                 350
Ala Ala Val Ala Gly Arg Asp Ala Thr Gly Arg Phe Thr Ala Gly Gln
        355                 360                 365
Pro Arg Arg Val Glu Leu Asp Ala Asp Ala Thr Ser Gly Ala Phe Tyr
    370                 375                 380
Ala Arg Tyr Arg Asp Gly Tyr Val Ser Gly Glu Pro Trp Pro Gly Ala
385                 390                 395                 400
Gly Pro Pro Pro Pro Gly Arg Val Leu Tyr Gly Gly Leu Gly Asp Ser
                405                 410                 415
```

```
Arg Pro Gly Leu Trp Gly Ala Pro Glu Ala Glu Glu Ala Arg Arg Arg
            420                 425                 430

Phe Glu Ala Ser Gly Ala Pro Ala Ala Val Trp Ala Pro Glu Leu Gly
        435                 440                 445

Asp Ala Ala Gln Gln Tyr Ala Leu Ile Thr Arg Leu Leu Tyr Thr Pro
    450                 455                 460

Asp Ala Glu Ala Met Gly Trp Leu Gln Asn Pro Arg Val Val Pro Gly
465                 470                 475                 480

Asp Val Ala Leu Asp Gln Ala Cys Phe Arg Ile Ser Gly Ala Ala Arg
                485                 490                 495

Asn Ser Ser Ser Phe Ile Thr Gly Ser Val Ala Arg Ala Val Pro His
            500                 505                 510

Leu Gly Tyr Ala Met Ala Ala Gly Arg Phe Gly Trp Gly Leu Ala His
        515                 520                 525

Ala Ala Ala Ala Val Ala Met Ser Arg Arg Tyr Asp Arg Ala Gln Lys
    530                 535                 540

Gly Phe Leu Leu Thr Ser Leu Arg Arg Ala Tyr Ala Pro Leu Leu Ala
545                 550                 555                 560

Arg Glu Asn Ala Ala Leu Thr Gly Ala Ala Gly Ser Pro Gly Ala Gly
                565                 570                 575

Ala Asp Asp Glu Gly Val Ala Ala Val Ala Ala Ala Ala Pro Gly Glu
            580                 585                 590

Arg Ala Val Pro Ala Gly Tyr Gly Ala Ala Gly Ile Leu Ala Ala Leu
        595                 600                 605

Gly Arg Leu Ser Ala Ala Pro Ala Ser Pro Ala Gly Gly Asp Asp Pro
    610                 615                 620

Asp Ala Ala Arg His Ala Asp Ala Asp Asp Asp Ala Gly Arg Arg Ala
625                 630                 635                 640

Gln Ala Gly Arg Val Ala Val Glu Cys Leu Ala Ala Cys Arg Gly Ile
                645                 650                 655

Leu Glu Ala Leu Ala Glu Gly Phe Asp Gly Asp Leu Ala Ala Val Pro
            660                 665                 670

Gly Leu Ala Gly Ala Arg Pro Ala Ser Pro Pro Arg Pro Glu Gly Pro
        675                 680                 685

Ala Gly Pro Ala Ser Pro Pro Pro Pro His Ala Asp Ala Pro Arg Leu
    690                 695                 700

Arg Ala Trp Leu Arg Glu Leu Arg Phe Val Arg Asp Ala Leu Val Leu
705                 710                 715                 720

Met Arg Leu Arg Gly Asp Leu Arg Val Ala Gly Gly Ser Glu Ala Ala
                725                 730                 735
```

```
Val Ala Ala Val Arg Ala Val Ser Leu Val Ala Gly Ala Leu Gly Pro
          740                 745                 750

Ala Leu Pro Arg Asp Pro Arg Leu Pro Ser Ser Ala Ala Ala Ala Ala
      755                 760                 765

Ala Asp Leu Leu Phe Asp Asn Gln Ser Leu Arg Pro Leu Leu Ala Ala
    770                 775                 780

Ala Ala Ser Ala Pro Asp Ala Ala Asp Ala Leu Ala Ala Ala Ala Ala
785                 790                 795                 800

Ser Ala Ala Pro Arg Glu Gly Arg Lys Arg Lys Ser Pro Gly Pro Ala
                805                 810                 815

Arg Pro Pro Gly Gly Gly Gly Pro Arg Pro Pro Lys Thr Lys Lys Ser
            820                 825                 830

Gly Ala Asp Ala Pro Gly Ser Asp Ala Arg Ala Pro Leu Pro Ala Pro
        835                 840                 845

Ala Pro Pro Ser Thr Pro Pro Gly Pro Glu Pro Ala Pro Ala Gln Pro
    850                 855                 860

Ala Ala Pro Arg Ala Ala Ala Ala Gln Ala Arg Pro Arg Pro Val Ala
865                 870                 875                 880

Val Ser Arg Arg Pro Ala Glu Gly Pro Asp Pro Leu Gly Gly Trp Arg
                885                 890                 895

Arg Gln Pro Pro Gly Pro Ser His Thr Ala Ala Pro Ala Ala Ala Ala
            900                 905                 910

Leu Glu Ala Tyr Cys Ser Pro Arg Ala Val Ala Glu Leu Thr Asp His
        915                 920                 925

Pro Leu Phe Pro Val Pro Trp Arg Pro Ala Leu Met Phe Asp Pro Arg
    930                 935                 940

Ala Leu Ala Ser Ile Ala Ala Arg Cys Ala Gly Pro Ala Pro Ala Ala
945                 950                 955                 960

Gln Ala Ala Cys Gly Gly Gly Asp Asp Asp Asp Asn Pro His Pro His
                965                 970                 975

Gly Ala Ala Gly Gly Arg Leu Phe Gly Pro Leu Arg Ala Ser Gly Pro
            980                 985                 990

Leu Arg Arg Met Ala Ala Trp Met  Arg Gln Ile Pro Asp  Pro Glu Asp
        995                 1000                1005

Val Arg  Val Val Val Leu Tyr  Ser Pro Leu Pro Gly  Glu Asp Leu
    1010                 1015                 1020

Ala Gly  Gly Gly Ala Ser Gly  Gly Pro Pro Glu Trp  Ser Ala Glu
    1025                 1030                 1035

Arg Gly  Gly Leu Ser Cys Leu  Leu Ala Ala Leu Ala  Asn Arg Leu
    1040                 1045                 1050

Cys Gly  Pro Asp Thr Ala Ala  Trp Ala Gly Asn Trp  Thr Gly Ala
```

1055 1060 1065

Pro Asp Val Ser Ala Leu Gly Ala Gln Gly Val Leu Leu Leu Ser
1070 1075 1080

Thr Arg Asp Leu Ala Phe Ala Gly Ala Val Glu Phe Leu Gly Leu
1085 1090 1095

Leu Ala Ser Ala Gly Asp Arg Arg Leu Ile Val Val Asn Thr Val
1100 1105 1110

Arg Ala Cys Asp Trp Pro Ala Asp Gly Pro Ala Val Ser Arg Gln
1115 1120 1125

His Ala Tyr Leu Ala Cys Glu Leu Leu Pro Ala Val Gln Cys Ala
1130 1135 1140

Val Arg Trp Pro Ala Ala Arg Asp Leu Arg Arg Thr Val Leu Ala
1145 1150 1155

Ser Gly Arg Val Phe Gly Pro Gly Val Phe Ala Arg Val Glu Ala
1160 1165 1170

Ala His Ala Arg Leu Tyr Pro Asp Ala Pro Pro Leu Arg Leu Cys
1175 1180 1185

Arg Gly Gly Asn Val Arg Tyr Arg Val Arg Thr Arg Phe Gly Pro
1190 1195 1200

Asp Thr Pro Val Pro Met Ser Pro Arg Glu Tyr Arg Arg Ala Val
1205 1210 1215

Leu Pro Ala Leu Asp Gly Arg Ala Ala Ala Ser Gly Thr Thr Asp
1220 1225 1230

Ala Met Ala Pro Gly Ala Pro Asp Phe Cys Glu Glu Glu Ala His
1235 1240 1245

Ser His Ala Ala Cys Ala Arg Trp Gly Leu Gly Ala Pro Leu Arg
1250 1255 1260

Pro Val Tyr Val Ala Leu Gly Arg Glu Ala Val Arg Ala Gly Pro
1265 1270 1275

Ala Arg Trp Arg Gly Pro Arg Arg Asp Phe Cys Ala Arg Ala Leu
1280 1285 1290

Leu Glu Pro Asp Asp Asp Ala Pro Pro Leu Val Leu Arg Gly Asp
1295 1300 1305

Asp Asp Gly Pro Gly Ala Leu Pro Pro Ala Pro Pro Gly Ile Arg
1310 1315 1320

Trp Ala Ser Ala Thr Gly Arg Ser Gly Thr Val Leu Ala Ala Ala
1325 1330 1335

Gly Ala Val Glu Val Leu Gly Ala Glu Ala Gly Leu Ala Thr Pro
1340 1345 1350

Pro Arg Arg Glu Val Val Asp Trp Glu Gly Ala Trp Asp Glu Asp
1355 1360 1365

Asp Gly Gly Ala Phe Glu Gly Asp Gly Val Leu
1370 1375

<210> 6
<211> 4220
<212> DNA
<213> Artificial Sequence

<220>
<223> Kozak-NLS-ODD-ICP4

<400> 6
aattcccagc ttgacatggc gcctaagaag aagaggaaga gatcatatgg tcgtaagaaa 60
cgtcgccaac gtcgccgaag atctaaccca ttttctactc aggacacaga tttagacttg 120
gagatgttag ctccctatat cccaatggat gatgacttcc agttacgttc cttcgatcag 180
ttgtcaccat tagaaagcag ttccgcaagc cctgaaagcg caagtcctca aagcacagtt 240
acagtattcc agcaggtacc aatggcgtcg gagaacaagc agcgccccgg ctccccgggc 300
cccaccgacg ggccgccgcc caccccgagc ccagaccgcg acgagcgggg ggccctcggg 360
tggggcgcgg agacggagga gggcggggac gaccccgacc acgaccccga ccaccccac 420
gacctcgacg acgcccggcg ggacgggagg gccccgcgg cgggcaccga cgccggcgag 480
gacgccgggg acgccgtctc gccgcgacag ctggctctgc tggcctccat ggtagaggag 540
gccgtccgga cgatcccgac gcccgacccc gcggcctcgc cgccccggac cccgcctttt 600
cgagccgacg acgatgacgg ggacgagtac gacgacgcag ccgacgccgc cggcgaccgg 660
gccccggccc ggggccgcga acgggaggcc ccgctacgcg gcgcgtatcc ggaccccacg 720
gaccgcctgt cgccgcgccc gccggcccag ccgccgcgga gacgtcgtca cggccggtgg 780
cggccatcgg cgtcatcgac ctcgtcggac tccgggtcct cgtcctcgtc gtccgcatcc 840
tcttcgtcct cgtcgtccga cgaggacgag gacgacgacg gcaacgacgc ggccgaccac 900
gcacgcgagg cgcgggccgt cgggcggggt ccgtcgagcg cggcgccggc agcccccggg 960
cggacgccgc cccgcccgg gccacccccc ctctccgagg ccgcgcccaa gccccgggcg 1020
gcggcgagga cccccgcggc ctccgcgggc cgcatcgagc gccgccgggc ccgcgcggcg 1080
gtggccggcc gcgacgccac gggccgcttc acggccgggc agccccggcg ggtcgagctg 1140
gacgccgacg cgacctccgg cgccttctac gcgcgctatc gcgacgggta cgtcagcggg 1200
gagccgtggc ccggcgccgg gcccccgccc ccggggcggg tgctgtacgg cggcctgggc 1260
gacagccgcc cgggcctctg gggggcgccc gaggcggagg aggcgcgacg ccggttcgag 1320
gcctcgggcg ccccggcggc cgtgtgggcg cccgagctgg gcgacgccgc gcagcagtac 1380
gccctgatca cgcggctgct gtacaccccg gacgcggagg ccatggggtg gctccagaac 1440
ccgcgcgtgg tccccgggga cgtggcgctg gaccaggcct gcttccggat ctcgggcgcc 1500
gcgcgcaaca gcagctcctt catcaccggc agcgtggcgc gggccgtgcc ccacctgggc 1560
tacgccatgg cggccggccg cttcggctgg ggcctggcgc acgcggcggc cgccgtggcc 1620
atgagccgcc gatacgaccg cgcgcagaag ggcttcctgc tgaccagcct gcgccgcgcc 1680
tacgcgcccc tgttggcgcg cgagaacgcg gcgctgacgg gggccgcggg gagccccggc 1740
gccggcgcag atgacgaggg ggtcgccgcc gtcgccgccg ccgcaccggg cgagcgcgcg 1800
gtgcccgccg ggtacggcgc cgcggggatc ctcgccgccc tggggcggct gtccgccgcg 1860
cccgcctccc ccgcgggggg cgacgacccc gacgccgccc gccacgccga cgccgacgac 1920
gacgccgggc gccgcgccca ggccggccgc gtggccgtcg agtgcctggc cgcctgccgc 1980
gggatcctgg aggcgctggc cgagggcttc gacggcgacc tggcggccgt cccggggctg 2040
gccggggccc ggcccgccag ccccccgcgg ccggagggac ccgcgggccc cgcttccccg 2100
ccgccgccgc acgccgacgc gccccgcctg cgcgcgtggc tgcgcgagct gcggttcgtg 2160
cgcgacgcgc tggtgctcat gcgcctgcgc ggggacctgc gcgtggccgg cggcagcgag 2220
gccgccgtgg ccgccgtgcg cgccgtgagc ctggtcgccg gggccctggg ccccgcgctg 2280
ccgcgggacc cgcgcctgcc gagctccgcg gccgccgccg ccgcggacct gctgtttgac 2340
aaccagagcc tgcgcccccct gctggcggcg gcggccagcg caccggacgc cgccgacgcg 2400
ctggcggccg ccgccgcctc cgccgcgccg cgggaggggc gcaagcgcaa gagtcccggc 2460
ccggcccggc cgcccggagg cggcggcccg cgacccccga agacgaagaa gagcggcgcg 2520
gacgcccccg gctcggacgc ccgcgccccc ctccccgcgc ccgcgccccc ctccacgccc 2580
ccggggcccg agcccgcccc cgcccagccc gcggcgcccc gggccgccgc ggcgcaggcc 2640
cgcccgcgcc ccgtggccgt gtcgcgccgg cccgccgagg gccccgaccc cctgggcggc 2700
tggcggcggc agcccccggg gcccagccac acggcggcgc ccgcggccgc cgccctggag 2760
gcctactgct ccccgcgcgc cgtggccgag ctcacggacc acccgctgtt ccccgtcccc 2820
tggcgaccgg ccctcatgtt tgacccgcgg gccctggcct cgatcgccgc gcggtgcgcc 2880

```
gggcccgccc ccgccgccca ggccgcgtgc ggcggcggcg acgacgacga taaccccccac   2940
ccccacgggg ccgccggggg ccgcctcttt ggccccctgc gcgcctcggg cccgctgcgc   3000
cgcatggcgg cctggatgcg ccagatcccc gaccccgagg acgtgcgcgt ggtggtgctg   3060
tactcgccgc tgccgggcga ggacctggcc ggcggcgggg cctcgggggg gccgccggag   3120
tggtccgccg agcgcggcgg gctgtcctgc ctgctggcgg ccctggccaa ccggctgtgc   3180
gggccggaca cggccgcctg ggcgggcaat tggaccggcg cccccgacgt gtcggcgctg   3240
ggcgcacagg gcgtgctgct gctgtccacg cgggacctgg ccttcgccgg ggccgtggag   3300
tttctggggc tgctcgccag cgccggcgac cggcggctca tcgtggtcaa caccgtgcgc   3360
gcctgcgact ggcccgccga cgggcccgcg gtgtcgcggc agcacgccta cctggcgtgc   3420
gagctgctgc ccgccgtgca gtgcgccgtg cgctggccgg cggcgcggga cctgcgccgc   3480
acggtgctgg cctcgggccg cgtgttcggc ccgggggtct tcgcgcgcgt ggaggccgcg   3540
cacgcgcgcc tgtaccccga cgcgccgccg ctgcgcctgt gccgcggcgg caacgtgcgc   3600
taccgcgtgc gcacgcgctt cggcccggac acgccggtgc ccatgtcccc gcgcgagtac   3660
cgccgggccg tgctgccggc gctggacggc cgggcggcgg cctcggggac caccgacgcc   3720
atggcgcccg gcgcgccgga cttctgcgag gaggaggccc actcgcacgc cgcctgcgcg   3780
cgctggggcc tgggcgcgcc gctgcggccc gtgtacgtgg cgctggggcg cgaggcggtg   3840
cgcgccggcc cggcccggtg gcgcgggccg cggagggact tttgcgcccg cgccctgctg   3900
gagcccgacg acgacgcccc cccgctggtg ctgcgcggcg acgacgacgg cccgggggcc   3960
ctgccgccgg cgccgcccgg gattcgctgg gcctcggcca cgggccgcag cggcaccgtg   4020
ctggcggcgg cgggggccgt ggaggtgctg ggggcggagg cgggcttggc cacgccccccg   4080
cggcgggaag ttgtggactg ggaaggcgcc tgggacgaag acgacggcgg cgcgttcgag   4140
ggggacgggg tgctgtaacg ggccgggacg gggcggggcg cttgtgagac ccgaagacgc   4200
aataaacggc aacaacctga                                                 4220
```

## Description

Technical Field

The present invention relates to a virus that comprises a gene encoding a fusion protein of an ODD and a protein essentially required for viral proliferation, a method for producing a virus that uses the virus, a virus or a viral vector that is obtainable by the method, and a pharmaceutical composition comprising the virus or viral vector for treating or preventing a disease characterized by a cell in a hypoxic state. Further, the present application claims the benefit of priority from Japanese Patent Application No. 2008-7179, filed on January 6, 2008, and the entire content of this Japanese Patent Application No. 2008-7179 is incorporated herein by reference.

Background Art

During the course of previous research, the inventors of the present invention modified herpes simplex virus type I (HSV-1) gene to enable the virus to only proliferate in cells in the case of having infected cells that express calponin gene, namely smooth muscle cells growing in leiomyoma, mesothelioma or sites of vascular stenosis, and as a result thereof, developed a method for expressing genes within infected cells and disrupting infected cells (Patent Document 1).

Cancer cells present in a hypoxic environment within tumors are unresponsive to radiotherapy or chemotherapy, and have the ability to metastasize to distant organs. In addition, cancer stem cells have been reported to be present in cell populations having self-replicating ability in hypoxic regions of bone marrow in acute myelogenous leukemia (Non-Patent Document 1). Moreover, the presence of cancer stem cells has also been recently reported in solid cancers such as breast cancer, brain tumors and colorectal cancer (Non-Patent Document 2).

In general, cancer stem cells are considered to be cancer cells that are resistant to therapy, are highly metastatic, and are able to self-replicate even in hypoxic environments (Non-Patent Document 3). Thus, the development of a treatment method is sought that enables cancer cells present in hypoxic regions such as cancer stem cells to be selectively disrupted.

Research has been conducted on a method for stably expressing arbitrary proteins under hypoxic conditions by using an oxygen-dependent degradation domain (ODD) that serves as a marker for protein degradation at normal oxygen partial pressure present in the amino acid sequence of transcription factor HIFIα, which stabilizes only in cells in a hypoxic state. For example, a method has been developed for inducing apoptosis in cancer cells by producing a fusion gene in which an ODD sequence has been added to the amino terminal of the apoptosis stimulating factor, caspase 3, and activating the caspase 3 only in cells in a hypoxic state, and a method has been developed for expressing diphtheria toxin protein (Non-Patent Documents 4 and 5). However, these methods not only target abnormal cells such as cancer cells, but target normal cells as well. In addition, since these methods involve direct administration of protein whose expression has been inhibited by an ODD sequence, there is the disadvantage of weak therapeutic effects since expression is unable to be sustained due to degradation and the like. Thus, there is a need to develop a method that allows obtaining specific and potent therapeutic effects in target cells. In addition, although adenovirus has previously been produced that is capable of replicating only in cells under hypoxic conditions by coupling E1A gene downstream to the promoter sequence, Hypoxia-Responsive Element (HRE), which responds to HIF1α, there is concern over toxic activity against normal cells due to insufficient on-off control of oxygen partial pressure-dependent promoter activity (Non-Patent Document 6).

Patent Document 1: Japanese Patent Application No. 2006-205006
Non-Patent Document 1: Sipkins D.A. et al. Nature 435, 969-973, 2005
Non-Patent Document 2: Ailles L.E. and Weissman I.L. Curr. Opin. Biotech. 18, 460-466, 2007
Non-Patent Document 3: De Toni F. et al. Oncogene 25, 3113-3122, 2006
Non-Patent Document 4: Harada H. et al. Cancer Res. 62, 2013-2018, 2002
Non-Patent Document 5: Koshikawa N. and Takenaga K. Cancer Res. 65, 11622-11630, 2005
Non-Patent Document 6: Cuevas Y. et al. Cancer Res. 61, 6877-6884, 2003

Disclosure of the Invention

Problems to be Solved by the Invention

An object of the present invention is to provide a virus for specifically injuring cells such as cancer stem cells in a hypoxic state, a viral vector that expresses more genes in cells in a hypoxic state, and preferably in cells having the ability to replicate in a hypoxic state, and a pharmaceutical composition comprising the virus and the viral vector.

Means for Solving the Problems

As a result of conducting extensive studies with the foregoing in view, the inventors of the present invention fused an oxygen-dependent degradation domain (ODD) with a protein essentially required for viral proliferation, and produced a virus that comprises a gene encoding the fusion protein. Although the fusion protein is degraded following recognition by the ubiquitin-proteasome system as a result of the fusion protein being modified by hydroxylation of a proline residue in the ODD sequence by proline hydroxylase in the case the virus has infected a cell at normal oxygen partial pressure, the fusion protein is not degraded under hypoxic conditions such as at an oxygen partial pressure of 10 mmHg or less. Thus, it was found that the virus does not proliferate in cells at normal oxygen partial pressure, but rather proliferates specifically in the case of having infected cells growing in a hypoxic state, that the gene carried by the virus is expressed, and that specific cytolytic action can be made to occur, thereby leading to completion of the present invention.

Namely, the present invention provides the inventions indicated in (1) to (16) below:

(1) a virus or viral vector, comprising: a gene encoding a fusion protein of an oxygen-dependent degradation domain (ODD) and a protein essentially required for viral proliferation;
(2) the virus or viral vector described in (1), wherein the protein essentially required for viral proliferation is selected from the group consisting of herpes virus ICP4, γ34.5, adenovirus E1A, E1B, retrovirus LTR R and U5;
(3) the virus or viral vector described in (2), wherein the protein essentially required for viral proliferation is ICP4;
(4) the virus or viral vector described in any of (1) to (3) which has a deficiency in ribonucleotide reductase (RR);
(5) a method for producing a virus or viral vector, comprising the steps of:

(a) infecting a cell with the virus described in any of (1) to (4);
(b) culturing the cell in a hypoxic state; and
(c) recovering the virus that has proliferated;

(6) a virus or viral vector, which is obtainable by the method described in (5);
(7) the virus or viral vector described in (6) which is of the strain d12.ODDΔRR;
(8) a pharmaceutical composition for treating or preventing a disease characterized by a cell having replicating ability in a hypoxic state, which comprises the virus or viral vector described in any of (1) to (4) or the virus or viral vector described in (6) or (7);
(9) the pharmaceutical composition described in (8), wherein the disease is selected from the group consisting of cancer, pulmonary fibrosis, pulmonary hypertension, and vascular stenosis in ischemic heart disease or ischemic brain disease;
(10) the pharmaceutical composition described in (9), wherein the disease is cancer;
(11) the pharmaceutical composition described in (10) which targets a cancer stem cell present in cancer;
(12) the pharmaceutical composition described in any of (8) to (11), wherein the hypoxic state is a state in which oxygen partial pressure is 10 mmHg or less;
(13) a method for treating or preventing a disease comprising: administering to a subject the virus or viral vector described in any of (1) to (4) or the virus or viral vector described in (6) or (7), wherein the disease is characterized by a cell in the subject having replicating ability in a hypoxic state;
(14) a use of the virus or viral vector described in any of (1) to (4) or the virus or viral vector described in (6) or (7) in manufacturing a medicament for treating or preventing a disease in a subject, wherein the disease is characterized by a cell in the subject having replicating ability in a hypoxic state;
(15) a fusion protein of an ODD and a protein essentially required for viral proliferation; and,
(16) a polynucleotide encoding the protein described in (15).

Effect of the Invention

Thus, according to the present invention, cells such as cancer stem cells in a hypoxic state, and particularly cells having the ability to replicate in a hypoxic state, can be specifically injured, thereby making it possible to effectively treat or prevent diseases such as cancer, pulmonary fibrosis, pulmonary hypertension or vascular stenosis in ischemic heart disease or ischemic brain disease.

Brief Description of the Drawings

FIG. 1 indicates expressions of EGFP, HIF1α and ICP4 after culturing human gastric cancer cell line AZP7a, transfected with a CMV-NLS-ODD-ICP4-IRES-EGFP-polyA DNA fragment, in RPMI medium containing 10% FBS at normal oxygen partial pressure ($O_2$: 20%) or low oxygen partial pressure ($O_2$: 1%) as observed with a fluorescence microscope;

FIG. 2 indicates plaque formation by HSV-1 virus in human gastric cancer cell line AZP7a, co-transfected with DNA of ICP4-deficient HSV-1 mutant strain d120 and a CMV-NLS-ODD-ICP4-IRES-EGFP-polyA DNA fragment, at normal oxygen partial pressure ($O_2$: 20%) or low oxygen partial pressure ($O_2$: 1%);

FIG. 3 indicates construction of d12.ODDΔRR;

FIG. 4 represents the proliferation ability of HSV-1 virus as total plaque area when Vero cells are infected with purified d12.ODDΔRR and cultured for 24 hours at normal oxygen partial pressure ($O_2$: 20%) or low oxygen partial pressure ($O_2$: 1%), and indicates that d12.ODDΔRR proliferated well under conditions of low oxygen partial pressure ($O_2$: 1%);

FIG. 5 is a Western blot indicating expression of ICP4 protein when human gastric cancer cell line AZP7a was infected with purified d12.ODDΔRR at a multiplicity of infection (MOI) of 0.1 and cultured for 48 hours at normal oxygen partial pressure ($O_2$: 20%) or low oxygen partial pressure ($O_2$: 1%), and indicates that ICP4 was only expressed at low oxygen partial pressure ($O_2$: 1%);

FIG. 6 shows the results of viral replication analysis indicating ganciclovir sensitivity when Vero cells were infected with a purified d12.ODDΔRR at an MOI of 0.01 and cultured for 26 hours at normal oxygen partial pressure ($O_2$: 20%) or low oxygen partial pressure ($O_2$: 1%);

FIG. 7 indicates immunohistochemical results showing expression of ICP4 protein and the presence of HSV-1 envelope antigen indicating proliferation of d12.ODDΔRR in a hypoxic region labeled with pimonidazole (injection of d12.ODDΔRR at $1 \times 10^7$ pfu) located in a tumor in which human gastric cancer cell line AZP7a was transplanted into the abdominal cavities of SCID mice;

FIG. 8 indicates antitumor effects of d12.ODDΔRR against a tumor in which cultured human mesothelioma cell line MSTO was transplanted subcutaneously into the backs of SCID mice (intratumoral injection of d12.ODDΔRR at 1 to $2 \times 10^3$ pfu/injection), and tumor volume was determined by quantifying activity of luciferase transfected into the tumor as a photon count by real-time in vivo imaging;

FIG. 9 indicates antitumor effects of d12.ODDΔRR against tumors in which primary-cultured human leiomyoma cells established from surgical specimens (n = 8) were transplanted subcutaneously into the backs of SCID mice (d12.ODDΔRR initially injected intratumorally at 1 to $2 \times 10^3$ pfu/injection, and subsequently injected 17 times at 3 to 4 day intervals) as compared with d12.CALPΔRR (d12.CALPΔRR initially injected intratumorally at 1 to $2 \times 10^4$ pfu/injection, and subsequently injected 17 times at 3 to 4 day intervals), with the left side of the drawing indicating changes in tumor volume, and the right side of the drawing indicating residual tumor cells present in the tumor mass on day 82 following the start of treatment as observed with calponin immunostaining; and

FIG. 10 shows the results of a viral replication analysis in which cultured GIST cells were divided into fractions positive and negative for the cancer stem cell surface marker, CD133, using AutoMACS Pro (Miltenyi), followed by culturing in Petri dishes and infecting with purified d12.ODDΔRR at MOI of 0.01 to 0.0001.

Best Mode for Carrying Out the Invention

The present invention in one embodiment relates to a virus or viral vector comprising a gene encoding a fusion protein of an ODD and a protein essentially required for viral proliferation. As was previously described, an ODD (oxygen-dependent degradation domain) refers to a domain that serves as a marker for protein degradation by the ubiquitin-proteasome system at normal oxygen partial pressure present in the amino acid sequence of transcription factor HIF1α, which stabilizes only in a hypoxic state. The amino acid sequence of the ODD is shown in SEQ ID NO: 1, while the nucleic acid sequence thereof is shown in SEQ ID NO: 2. The ODD used in the present invention is composed of an amino acid sequence in which one or several amino acids have been deleted, substituted or added in the amino acid sequence indicated in SEQ ID NO: 1, and contains a nuclear localization signal (NLS) composed of 23 amino acids and the amino acid proline, which functions as a marker of protein degradation, on the amino terminal thereof. That amino acid sequence is shown in SEQ ID NO: 3. In addition, the nucleic acid sequence encoding the NLS-ODD sequence used in the present invention contains a Kozak consensus sequence on the 5' terminal thereof. That nucleic acid sequence is shown in SEQ ID NO: 4.

The protein essentially required for viral proliferation includes protein not only essentially required for viral proliferation, but also for replication of viral vector and/or viral nucleic acids. Various proteins are known as such proteins, and examples include, but are not limited to HSV-1 ICP4 and γ34.5, adenovirus E1A and E1B, retrovirus LTR R and U5, and herpes simplex virus ribonucleotide reductase (RR). The amino acid sequences of these proteins are known. The protein essentially required for viral proliferation used in the present invention comprises a protein that is composed of an amino acid sequence in which one or several, such as 9, 8, 7, 6, 5, 4, 3 or 2, amino acids have been deleted, substituted or added in the natural amino acid sequence, and that retains the function of the protein. Since the virus or viral vector of the present invention is used to injure infected cells by being replicated within the infected cells, the virus is preferably a replicative virus. In addition, the virus may be a DNA virus or an RNA virus. The mechanism by which infected cells are injured includes, for example, direct cytolysis due to viral proliferation, induction of apoptosis of virus-infected cells, activation of the immune system and the like.

The fusion protein of an ODD and a protein essentially required for viral proliferation is a protein in which these two proteins exist as a single polypeptide and are fused so as to retain the functions of each protein. A person with ordinary skill in the art is able to produce such fusion protein based on the sequences of the ODD and the protein essentially required for viral proliferation. Since such fusion protein is able to be degraded incidental to degradation by the ubiquitin-proteasome system of ODD, the presence and/or amount of the fusion protein can be controlled dependent on oxygen partial pressure of infected cells by using the virus or viral vector of the present invention. Preferably, the virus or viral vector of the present invention can be used as a vector that allows the expression of an arbitrary exogenous gene by coupling that gene through an internal ribosomal entry site (IRES) (U.S. Patent No. 4937190) downstream of a gene encoding the sequence of the protein essentially required for proliferation, and allows expression of a greater number of carried genes in cells in a hypoxic state, preferably in cells that have the ability to replicate in a hypoxic state, and for example, cancer stem cells.

Preferably, the virus or viral vector of the present invention has a deficiency in ribonucleotide reductase (RR). This deficiency refers to a state in which the function of RR has been lost, and is caused by, for example, deletion, insertion or mutation of a nucleotide in a gene encoding RR. RR is an enzyme that functions during the course of DNA synthesis, and is produced in large amounts in actively growing cells such as cancer cells. Thus, a deficiency in RR causes replication of the virus of the present invention to be carried out more frequently in actively growing cells such as cancer cells than in normal cells. In addition, as was previously described, since cells resistant to existing treatment methods, such as cancer stem cells, exist in a hypoxic state and have the ability to self-replicate, the virus or viral vector of the present invention is able to specifically injure growing cells and/or self-replicating cells in a hypoxic state, including cancer stem cells, as a result of having an ODD and being deficient in RR.

Preferably, the virus or viral vector of the present invention does not contain a gene encoding a protein essentially required for viral proliferation, such as a gene encoding RR, other than a gene encoding the protein essentially required for viral proliferation contained in the fusion protein. Virus replication can be controlled according to oxygen partial pressure more favorably by not containing such a gene.

In another embodiment, the present invention relates to a method for producing a virus or viral vector that comprises:

(1) infecting a cell with the above-mentioned virus of the present invention;
(2) culturing the cell in a hypoxic state; and,
(3) recovering the virus that has proliferated. Introduction of viral genes of the present invention into cells is carried out by ordinary virus infection. Cells infected by the virus may be any cells provided that the virus is able to replicate within the cells.

A hypoxic state refers to a state in which oxygen partial pressure is lower than the oxygen partial pressure of the normal proliferation environment of the virus or lower than the normal oxygen partial pressure within a cell, and for example refers to a state in which oxygen partial pressure is about 20 mmHg or less (or about 2% or less as oxygen partial pressure), and preferably a state in which oxygen partial pressure is about 10 mmHg or less (or about 1% or less as oxygen partial pressure). The fusion protein of ODD and the protein essentially required for viral proliferation is stabilized by culturing virus-infected cells in such a hypoxic state, and as a result, viral proliferation is begun, increased or accelerated. Culturing conditions other than a hypoxic state are suitably selected dependent on, for example, the cells infected by the virus.

The virus that has proliferated is recovered from the culture supernatant according to means or methods known among persons with ordinary skill in the art. The resulting virus can be infected into target cells in a hypoxic state, and preferably target cells that have the ability to replicate in a hypoxic state, and be allowed to proliferate for a fixed period of time enabling it to specifically injure the cells, or the virus or viral vector of the present invention can be obtained in a culture supernatant or in a supernatant obtained by lysing the virus-infected cells and centrifuging.

In another embodiment, the present invention relates to a virus or viral vector that is obtainable by a method for producing the above-mentioned virus or viral vector of the present invention. The virus or viral vector of the present invention efficiently proliferates in cells in a hypoxic state, and preferably in cells that have the ability to replicate in a hypoxic state. In addition, in the case the virus used in the method for producing the virus or viral vector of the present invention is HSV-1 deficient in RR, the virus or viral vector of the present invention is able to proliferate more favorably in actively growing cells such as cancer cells. Preferably, the virus or viral vector of the present invention is of strain d12.ODDΔRR. This strain d12.ODDΔRR will be described in detail herein-below. In addition, the virus or viral vector of the present invention is obtainable by infecting cells with the virus or viral vector of the present invention and causing viral nucleic acids to be replicated within the cells.

In a further embodiment, the present invention relates to cells that have been infected or introduced with the virus or viral vector of the present invention as previously described. Infection or introduction of the virus or viral vector of the present invention into cells can be carried out using any known methods. Cells that have been infected or introduced with the virus or viral vector of the present invention can be used to induce an immune response to the cells by being injected into the body.

In a still further embodiment, the present invention relates to a pharmaceutical composition for treating or preventing a disease characterized by cells being in a hypoxic state, and preferably cells having a replicating ability in a hypoxic state, comprising: the above-mentioned virus or viral vector of the present invention. Although previously defined, a hypoxic state preferably refers to a state in which oxygen partial pressure is about 10 mmHg or less (or about 1% or less as oxygen partial pressure). Examples of diseases characterized by cells having the ability to replicate in a hypoxic state include, but are not limited to, all forms of cancer, pulmonary fibrosis, pulmonary hypertension and vascular stenosis in ischemic heart disease or ischemic brain disease. In the case of cancer, for example, cells such as cancer stem cells have the ability to replicate in a hypoxic state. Since only these cells are considered to have the ability to allow cancer to recur or metastasize and be resistant to radiotherapy and chemotherapy, use of the pharmaceutical composition of the present invention enables diseases such as cancer that are resistant to existing treatment methods to be treated, and enables prevention of metastasis thereof.

The virus or viral vector contained in the pharmaceutical composition of the present invention is characterized by replicating or proliferating in cells in a hypoxic state, and preferably in cells that have the ability to replicate in a hypoxic state. Thus, the pharmaceutical composition comprising the virus or viral vector of the present invention is able to target only cells in a hypoxic state. More specifically, in the case a virus is contained in the pharmaceutical composition of the present invention, the pharmaceutical composition is able to injure only cells in a hypoxic state as a result of the virus proliferating in those cells. In the case a viral vector is contained in the pharmaceutical composition of the present invention, a specific gene can be expressed only in cells in a hypoxic state. Since the viral vector is preferably replicated, the amount of gene expressed in the cells in a hypoxic state is sustained at a high level.

Moreover, in the case the virus or viral vector contained in the pharmaceutical composition of the present invention has a deficiency in RR, the pharmaceutical composition of the present invention is able to more specifically injure actively growing cancer cells, and preferably cells such as cancer stem cells having the ability to self-replicate.

The pharmaceutical composition of the present invention may also contain various components such as a carrier, excipient or additive in addition to containing the virus or viral vector of the present invention. The components preferably increase infection efficiency of the virus or viral vector, and are suitably selected depending on various factors such as the disease, state of the target cells to be treated, or the administration mode. The amount and number of administrations of the virus or viral vector contained in the pharmaceutical composition of the present invention are suitably selected depending on the disease, target cells, subject status, administration mode and the like. In addition, known therapeutic agents of a disease to be treated or prevented may also be used in the pharmaceutical composition of the present invention or concomitantly with the pharmaceutical composition of the present invention.

In a yet further embodiment, the present invention relates to a method for treating and/or preventing a disease characterized by a cell of a subject being in a hypoxic state, comprising: administrating the pharmaceutical composition of the present invention to the subject. A known treatment method of a disease to be treated or prevented may be used concomitantly in the therapeutic and/or preventive method of the present invention.

In a further embodiment, the present invention relates to a use of the virus or viral vector of the present invention in manufacturing a medicament for treating and/or preventing a disease characterized by a cell being in a hypoxic state.

In another embodiment, the present invention relates to a fusion protein of an ODD and a protein essentially required for viral proliferation. The amino acid sequence of a fusion protein in which the protein essentially required for viral proliferation is HSV-1 ICP4 is shown in SEQ ID NO: 5. The fusion protein encompasses a protein which is composed of an amino acid sequence in which one or several, such as 9, 8, 7, 6, 5, 4, 3 or 2, of amino acids have been deleted, substituted or added in the amino acid sequence shown in SEQ ID NO: 5, and which has the function of the fusion protein, namely the function of the fusion protein of an ODD and a protein essentially required for viral proliferation. The fusion protein of the present invention can be produced according to a known method such as genetic recombination. The fusion protein of the present invention can be used to label a hypoxic region in cells in vivo, by introducing into the cells in advance.

In still another embodiment, the present invention relates to a polynucleotide that encodes the above-mentioned fusion protein of an ODD and a protein essentially required for viral proliferation. The nucleic acid sequence that encodes a fusion protein in which the protein essentially required for viral proliferation is HSV-1 ICP4 is shown in SEQ ID NO: 6.

Although the following provides a specific and detailed explanation of the present invention by indicating the following examples thereof, the examples should not be understood to limit the present invention.

Example 1

Production of Virus

A polypeptide composed of 57 proteins containing the 564th proline residue, which is the ubiquitin-proteasome recognition site within the ODD (oxygen-dependent degradation domain) of HIF1$\alpha$, which is an amino acid sequence serving as a marker of protein degradation at normal oxygen atmospheric pressure, was added to the amino terminal of ICP4, which is a transcription factor essentially required for the initiation of HSV-1 replication. Moreover, a nuclear localization signal composed of 23 amino acids was added to the amino terminal thereof.

ICP4 gene was PCR-amplified using a 4085-bp blunt end SalI-MseI fragment (provided by Dr. Hayward of John Hopkins School of Medicine) derived from pGH108 (J. Virol., 56, 558-570, 1985) containing the coding region thereof, from the initiation codon to a PvuII site, and a 261-bp DNA encoding a Kozak sequence (aattcccagcttgac), a sequence of 23 amino acids serving as a nuclear localization signal, and 57 ODD sequences, was coupled to the 5' terminal thereof. A PvuII-MseI fragment of ICP4 was coupled to the 3' side thereof to construct an ODD-fused ICP4 gene (4221 bp). Moreover, an approximately 9-kb CMV-NLS-ODD-ICP4-IRES-LacZ-polyA fragment was constructed in which a CMV promoter (588 bp) was coupled to the upstream side thereof, while a LacZ-polyA sequence (3.3 kb) derived from E. coli was coupled to the downstream side thereof through IRES (585 bp). Moreover, a CMV-NLS-ODD-ICP4-IRES-EGFP-polyA fragment was also constructed in which LacZ-polyA was substituted to EGFP-polyA.

First, the CMV-NLS-ODD-ICP4-IRES-EGFP-polyA fragment was co-transfected with a drug resistance gene expression vector, pSV2neo, into a sub-confluent single-layer culture of human gastric cancer cell line AZP7a (provided by Dr. Nishimori of the Sapporo Medical University) in a 6-well tissue culture plate ($2.5 \times 10^5$ /well) using Lipofectamine® (Gibco/BRL) in accordance with the manufacturer's protocol. G418-resistant clones of the gastric cancer cells that constitutively expressed EGFP were then selected using a method well known among persons with ordinary skill in the art. Whether or not expression of ICP4 protein changes depending on oxygen partial pressure was then examined using a fluorescence microscope under conditions of normal oxygen partial pressure ($O_2$: 20%) and low oxygen partial pressure ($O_2$: 1%) using the gastric cancer cell clones. Both EGFP and ICP4 were confirmed to be expressed within the same cells only under hypoxic conditions (FIG. 1). Although EGFP was expressed under both conditions of normal oxygen partial pressure ($O_2$: 20%) and low oxygen partial pressure ($O_2$: 1%), HIF1$\alpha$ and ICP4 were expressed only in cells cultured under conditions of low oxygen partial pressure ($O_2$: 1%). Expression of ODD-ICP4 fusion protein was confirmed to be controlled according to the oxygen partial pressure of the cells (FIG. 1). Moreover, the CMV-NLS-ODD-ICP4-IRES-EGFP-polyA fragment and HSV-1 variant d120 viral DNA deficient in ICP4 were co-transfected into AZP7a cells in accordance with the manufacturer's protocol under conditions of normal oxygen partial pressure ($O_2$: 20%) and low oxygen partial pressure ($O_2$: 1%), and the number of plaques was counted after 48 hours. Plaques were confirmed to only have been formed under conditions of low oxygen partial pressure ($O_2$: 1%) (FIG. 2). Plaque formation, which indicates viral proliferation, was observed to a much greater degree in the case of culturing at low oxygen partial pressure ($O_2$: 1%). Not only the expression of ODD-ICP4 fusion protein (FIG. 1 and Western blot), but also the function thereof were confirmed to be controlled according to the oxygen partial pressure of the cells.

The CMV-NLS-ODD-ICP4-IRES-LacZ-polyA fragment was inserted into an StuI site of pKpX2 (J. Virol., 62, 196-205, 1998) (provided by Dr. Weller of Connecticut University), which contains a 2.3 kb sequence of the 5' side of a gene (UL39) that encodes RR, which is an enzyme essentially required for replication of viral DNA, followed by linearization by digesting with XhoI (in which the XbaI site further towards the 5' side of the RR sequence on the 5' side of pKpX2 and the HindIII site further towards the 3' side of the RR sequence on the 3' were substituted with XhoI) to construct an 11.3-kb UL39-CMV-NLS-ODD-ICP4-IRES-LacZ-polyA-UL39 homologous recombination vector from which pUC 19 sequence is removed. This was then co-transfected with HSV-1 variant d120 viral DNA deficient in ICP4 into a sub-confluent, single-layer culture of VeroE5 cells constitutively expressing ICP4 in a 6-well tissue culture plate ($2.5 \times 10^5$ /well) using Lipofectamine® (Gibco/BRL) in accordance with the manufacturer's protocol. 1 ml of 20% DMEM culture medium was added 3 hours after transfection followed by culturing in the above-mentioned culture medium (10% FBS/DMEM) containing 0.5 mg/ml of 4-hydroxymethyl benzoic acid (HMBA) until 96 hours after transfection. Following confirmation of plaque formation, the cells were cultured for 24 hours in 10% FBS/DMEM not containing HMBA. The cells were then suspended in cold virus buffer (20 mM Tris-HC1 containing 150 mM NaCl, pH: 7.5) at 500 $\mu$l/well and placed in frozen storage.

Freezing and thawing treatment combined with ultrasonic treatment (3 times for 30 seconds each) were carried out three times to dissolve the above-mentioned suspension. The dissolved suspension was then stepwise-diluted and infected into sub-confluent, single-layer cultured VeroE5 cells in a 96-well tissue culture plate. Following infection, the cells were cultured for 96 hours in 1% FBS/DMEM containing 100 $\mu$l of 11.3 $\mu$g/ml human IgG (Jackson ImmunoResearch Laboratories) per well. VeroE5 single-layer cultured cells from those wells in which plaque formation was able to be confirmed were suspended in 100 $\mu$l of the above-mentioned culture medium, and 6 $\mu$l of the resulting suspension was used to measure $\beta$-galactosidase enzyme activity using 5-bromo-4-chloro-3-indolyl-$\beta$-D-galactopyranoside (X-gal) as the substrate and using a β galactosidase enzyme assay system (Promega). Suspensions of VeroE5 cells from wells positive for β-galactosidase enzyme activity were centrifuged for 5 minutes at 5000 rpm, and the resulting pellets were suspended in cold virus buffer at 100 μl/well. Similar measurement of limited dilution infection/β-galactosidase enzyme activity using a 96-well tissue culture plate was repeated two more times using VeroE5 cells to purify the recombinant virus d12.ODDΔRR as a single plaque (FIG. 3). The recombinant virus d12.ODDΔRR is maintained and managed by the inventors of the present invention.

Vero cells were infected in 10 to 20 150 $cm^2$ tissue culture flasks (T-150, Iwaki Glass), and after culturing for 48 hours in VP-SMF serum-free medium (Invitrogen), separated cells were centrifuged for 45 minutes at 42000 g and recovered in the sediment fraction to prepare the virus. The cells were then suspended in 10 ml of cold virus buffer (20 mM Tris-HCl containing 150 mM NaCl, pH: 7.5). Freezing and thawing treatment combined with ultrasonic treatment (3 times for 30 seconds each) were carried out three times to dissolve the above-mentioned cells, followed by centrifuging for 5 minutes at 4°C and 500 × g, and further centrifuging the supernatant for 45 minutes at 4°C and 26100 × g. The resulting pellet was suspended in cold virus buffer, a plaque assay was carried out using Vero cells under conditions of normal oxygen partial pressure ($O_2$: 20%) and low oxygen partial pressure ($O_2$: 1%), and proliferating activity of the purified d12.ODDΔRR, sensitivity of its cell-injuring action to oxygen partial pressure, and its titer were determined. d12.ODDΔRR proliferated more actively and demonstrated cell-injuring activity under conditions of low oxygen partial pressure ($O_2$: 1%) (FIG. 4).

Immunoblot Analysis of ICP4 Expression

AZP7a cells were respectively infected with d12.ODDΔRR to a multiplicity of infection (MOI) of 0.1 or only virus buffer under conditions of normal oxygen partial pressure ($O_2$: 20%) and low oxygen partial pressure ($O_2$: 1%), followed by recovery of the cells after culturing for 48 hours. An equal amount of protein was applied to SDS-PAGE gel electrophoresis and transferred to a nitrocellulose membrane (Bio-Rad). After blocking the membrane for 2 hours at room temperature using 5% skim milk (Difco Laboratories), the protein was incubated overnight at 4°C with anti-ICP4 antibody (Goodwin Institute for Cancer Research, dilution factor: 1:500). A larger amount of ICP4 protein was expressed under conditions of low oxygen partial pressure ($O_2$: 1%) (FIG. 5). Expression of ICP4 was not observed with virus buffer only even after addition of AZP7a.

Virus Replication Analysis Indicating Ganciclovir Sensitivity of d12.ODDΔRR

Vero cell line was cultured in a 24-well culture plate at $5 \times 10^4$ cells/well, and after infecting with d12.ODDΔRR virus at an MOI of 0.01, various concentrations (0 to 1 μg/ml) of ganciclovir (Wako Pure Chemical Industries) in 1% FBS/DMEM were added followed by culturing for 26 hours. After fixing the cells with 10% formalin PBS, the resulting plaques were stained with X-Gal and counted. Proliferation of d12.ODDΔRR was inhibited concentration-dependently by addition of ganciclovir under both conditions of normal oxygen partial pressure ($O_2$: 20%) and low oxygen partial pressure ($O_2$: 1%). The results are shown in FIG. 6.

(In Vivo Treatment and Histological Analysis)

AZP7a cells were injected into the peritoneal cavities of 6-week-old, female severe combined immunodeficiency (SCID) mice (Nippon Clea), and MSTO human malignant mesothelioma cells (ATCC-, CRL-2081), primary-cultured human leiomyoma cells and MCF7 human breast cancer cells (ATCC, HTB-22) were injected subcutaneously into the trunk to establish tumors in the mice. The MSTO cells were transfected with luciferase gene pGL4.13 (Promega), and clones demonstrating the highest chemiluminescence intensity and growth rate were selected. Among the cloned cells, tumor masses of the mesothelioma measuring 4 to 5 mm square were subcutaneously transplanted from those that had become established beneath the skin on the backs of the SCID mice to backs of 6-week-old, female SCID mice. The tumors grew to about 6 to 7 mm in diameter (50 to 70 $mm^3$) at 30 days after being transplanted to the SCID mice. 50 μl (per 100 $mm^3$ of tumor volume) of virus suspension containing $1 \times 10^7$ pfu of d12.ODDΔRR, or an equal volume of virus buffer, were injected intraperitoneally or intratumorally using a 30 gauge needle. The procedure was then repeated in exactly the same manner. Tumor diameter was measured at prescribed times after injection, and tumor volume was calculated using the following formula:

$$(\text{tumor volume}) = 0.53 \times (\text{length}) \times (\text{width})^2$$

In the case of MSTO, luciferin (Sigma Chemicals) was injected intraperitoneally, and in use of a high-sensitivity CCD camera the intensity of chemiluminescence from tumor cells beneath the skin of the back was measured employing a real-time in vivo imaging system (Berthold). d12.ODDΔRR demonstrated remarkable antitumor effects against each of the subcutaneously transplanted human malignant mesothelioma, leiomyoma and breast cancer cells by direct injection. The immunohistochemical results for human gastric cancer cells are shown in FIG. 7, while the treatment results for human malignant mesothelioma and leiomyoma are shown in FIGS. 8 and 9. Antitumor effects in a treatment group injected with d12.ODDΔRR a total of seven times on the prescribed days indicated with arrows were clearly evident in comparison with a control group injected with virus buffer only (FIG. 8). In contrast to residual tumor cells not being observed in tumors treated with d12.ODDΔRR, residual tumor cells were observed in tumors treated with d12.CALPΔRR (FIG. 9).

Mice in which tumors were present were sacrificed on prescribed numbers of days following completion of administration of d12.ODDΔRR at $1 \times 10^7$ pfu/100 $mm^3$ of tumor volume for the purpose of histological research. The subcutaneous tumors were excised and fixed overnight at 4°C in PBS containing 1 mM $MgCl_2$ using 2% paraformaldehyde and 0.5% glutaraldehyde. Continuing, the tumors were immersed for 3 hours at 37°C in a substrate solution containing X-gal (1 mg/ml), 5 mM $K_3Fe(CN_6)$, 5 mM $K_4Fe(CN_6)$ and 1 mM $MgCl_2$ in PBS, followed by washing with PBS containing 3% DMSO. Immunohistochemistry consisted of fixing the specimens in Bouin's solution (15% (v/v) saturated picric acid solution, 1.65% (v/v) formalin and 1% (v/v) acetic acid/PBS) followed by embedding in paraffin. A section having a thickness of 4 μm was placed on a microslide coated with poly-L-lysine followed by treatment in xylene and stepwise dehydration with alcohol, and in order to block intrinsic peroxidase, the section was immersed in a solution of 70% methanol and $H_2O_2$. Subsequently, antigen was recovered in 10 mM citrate buffer (pH 7.0) using an autoclave for 10 minutes at 121°C. The section was then incubated for 1 hour at room temperature using 1% (v/v) goat serum/PBS, followed by washing with PBS and incubating overnight at 4°C in 2% (w/v) BSA/PBS using the previously described anti-ICP4 antibody or anti-envelope antibody (Quartett). The section was then washed five times with 0.005% (v/v) Tween20/PBS followed by incubating for 1 hour at room temperature in 2% (w/v) BSA/PBS using biotinated goat anti-rabbit IgG (Tago Immunologicals), and further incubating for 30 minutes at room temperature using avidin-biotin-horseradish peroxidase conjugate (Vector Laboratories). After washing with 0.005% (v/v) Tween20/PBS, the final reaction product was visualized with diaminobentidine (Wako Chemicals), and the section was counter-stained with hematoxylin. A tissue specimen treated with goat serum was used as a control to observe non-specific staining. Detection of hypoxic regions of tissue was carried out using the Hypoxyprobe-1 Kit (Natural Pharmacia International) in accordance with the manufacturer's protocol. In an SCID mouse intraperitoneal transplant model of human gastric cancer cell line AZP7a, proliferation of intraperitoneally injected d12.ODDΔRR in coordination with hypoxic regions within tumors labeled with pimonidazole and expression of ICP4 protein were confirmed (FIG. 7).

After dividing cultured GIST cells into fractions positive and negative for CD133, which is a cell surface marker of cancer stem cells, using AutoMACS Pro (Miltenyi), the cells were cultured in Petri dishes and infected with purified d12.ODDΔRR at MOI of 0.01 to 0.0001 followed by analyzing virus replication, the results of which are shown in FIG. 10. d12.ODDΔRR proliferated more in CD133-positive cells and demonstrated potent cell-injuring action.

Industrial Applicability

Since a virus or viral vector comprising a gene encoding a fusion protein of an ODD and a protein essentially required for viral proliferation, and a pharmaceutical composition comprising the same, are provided by the present invention, the present invention can be used in the development and production of therapeutic agents in the field of pharmaceuticals and the like, such as those for treatment of cancer under hypoxic conditions and resistant to radiotherapy and chemotherapy, and preferably cancer stem cells.

Sequence Listing Free Text

SEQ ID NO: 1: ODD
SEQ ID NO: 2: ODD
SEQ ID NO: 3: NLS-ODD
SEQ ID NO: 4: Kozak-NLS-ODD
SEQ ID NO: 5: ODD-ICP4 fusion protein
SEQ ID NO: 6: Kozak-NLS-ODD-ICP4

## Claims

1. A virus or viral vector, comprising: a gene encoding a fusion protein of an oxygen-dependent degradation domain (ODD) and a protein essentially required for viral proliferation.

2. The virus or viral vector according to claim 1, wherein the protein essentially required for viral proliferation is selected from the group consisting of herpes virus ICP4, γ34.5, adenovirus E1A, E1B, retrovirus LTR R and U5.

3. The virus or viral vector according to claim 2, wherein the protein essentially required for viral proliferation is ICP4.

4. The virus or viral vector according to any one of claims 1 to 3, which has a deficiency in ribonucleotide reductase (RR).

5. A method for producing a virus or viral vector, comprising the steps of:

   (a) infecting a cell with the virus according to any one of claims 1 to 4;
   (b) culturing the cell in a hypoxic state; and
   (c) recovering the virus that has proliferated.

6. A virus or viral vector, which is obtainable by the method according to claim 5.

7. The virus or viral vector according to claim 6, which is of the strain d12.ODDΔRR.

8. A pharmaceutical composition for treating or preventing a disease **characterized by** a cell having replicating ability in a hypoxic state, which comprises the virus or viral vector according to any one of claims 1 to 4 or the virus or viral vector according to claim 6 or 7.

9. The pharmaceutical composition according to claim 8, wherein the disease is selected from the group consisting of cancer, pulmonary fibrosis, pulmonary hypertension, and vascular stenosis in ischemic heart disease or ischemic brain disease.

**10.** The pharmaceutical composition according to claim 9, wherein the disease is cancer.

**11.** The pharmaceutical composition according to claim 10, which targets a cancer stem cell present in cancer.

**12.** The pharmaceutical composition according to any one of claims 8 to 11, wherein the hypoxic state is a state in which oxygen partial pressure is 10 mmHg or less.

**13.** A method for treating or preventing a disease comprising: administering to a subject the virus or viral vector according to any one of claims 1 to 4 or the virus or viral vector according to claim 6 or 7, wherein the disease is **characterized by** a cell in the subject having replicating ability in a hypoxic state.

**14.** A use of the virus or viral vector according to any one of claims 1 to 4 or the virus or viral vector according to claim 6 or 7 in manufacturing a medicament for treating or preventing a disease in a subject, wherein the disease is **characterized by** a cell in the subject having replicating ability in a hypoxic state.

**15.** A fusion protein of an ODD and a protein essentially required for viral proliferation.

**16.** A polynucleotide encoding the protein according to claim 15.

FIG. 1

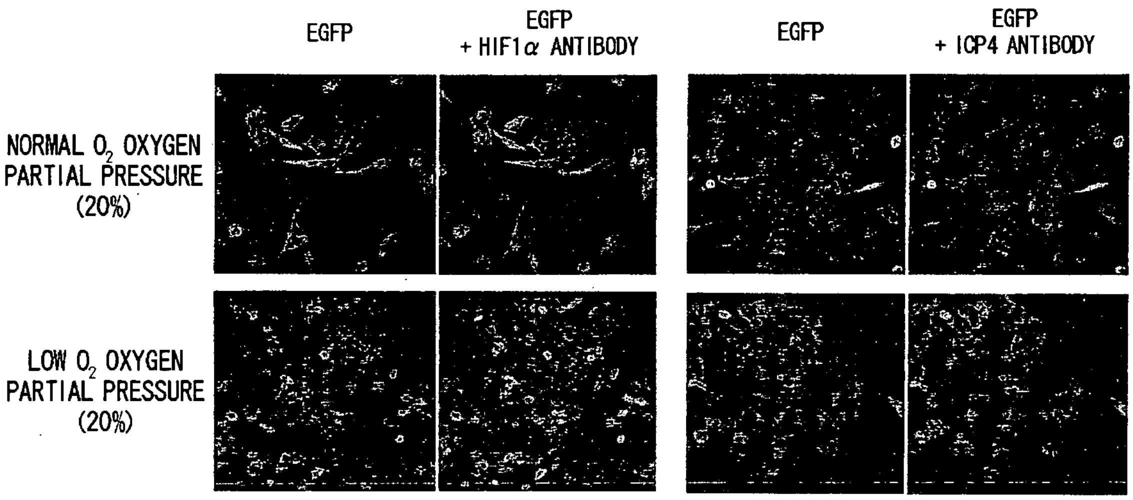
EGFP
EGFP + HIF1α ANTIBODY
EGFP
EGFP + ICP4 ANTIBODY
NORMAL O2 OXYGEN PARTIAL PRESSURE (20%)
LOW O2 OXYGEN PARTIAL PRESSURE (20%)

Fig. 2

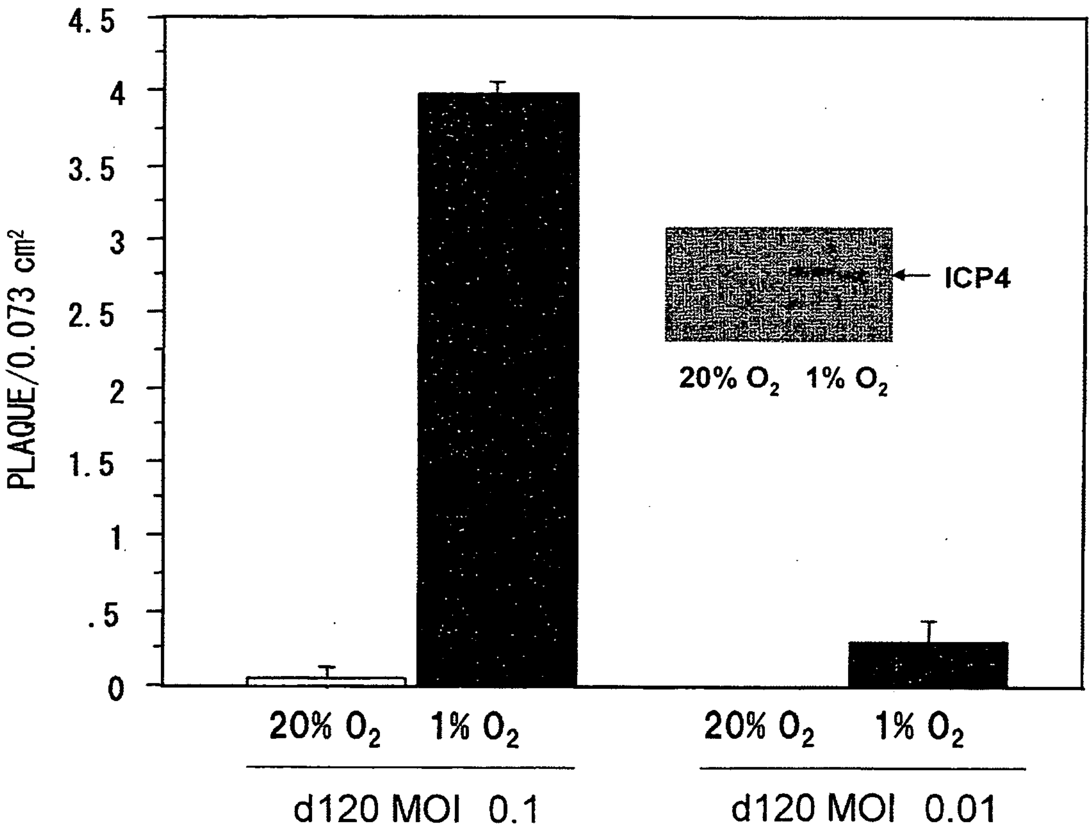
4.5
4
3.5
3
2.5
2
1.5
1
.5
0
PLAQUE/0.073 cm2
ICP4
20% O2 1% O2
20% O2 1% O2
d120 MOI 0.1
20% O2 1% O2
d120 MOI 0.01

FIG. 3

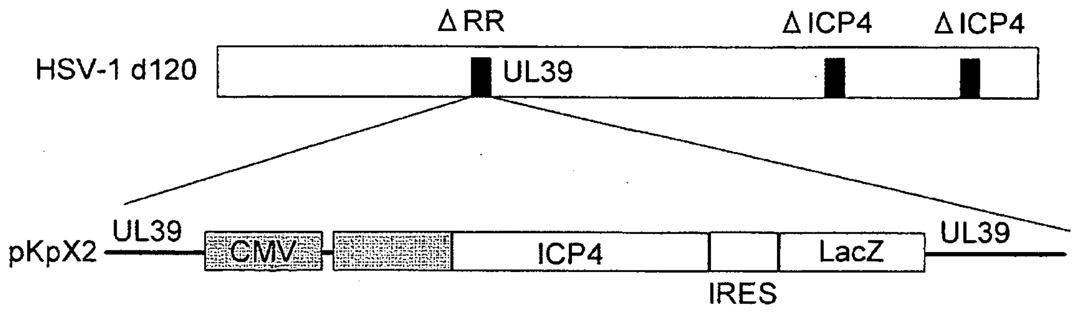
ΔRR
ΔICP4
ΔICP4
HSV-1 d120
UL39
pKpX2
UL39
CMV
ICP4
LacZ
UL39
IRES

FIG. 4

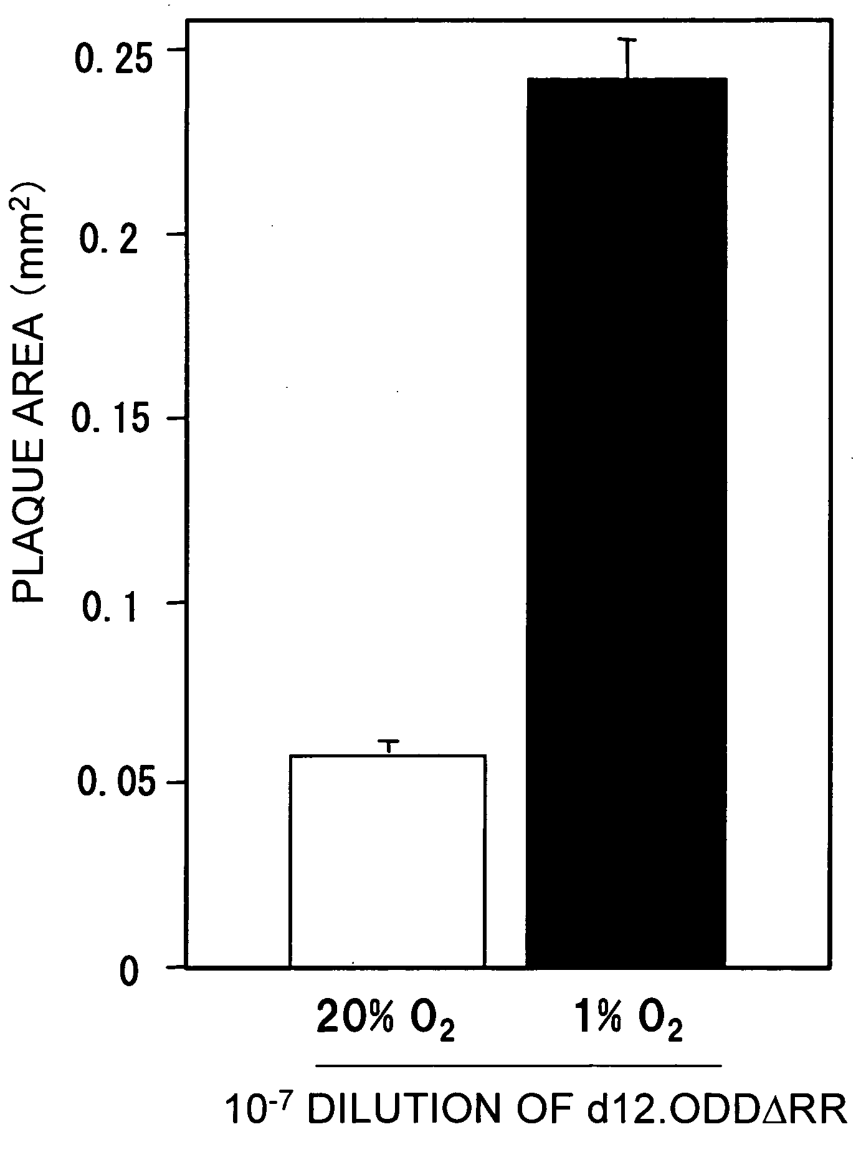
0.25
0.2
0.15
0.1
0.05
0
PLAQUE AREA (mm²)
20% O₂
1% O₂
10⁻⁷ DILUTION OF d12.ODDΔRR

FIG. 5

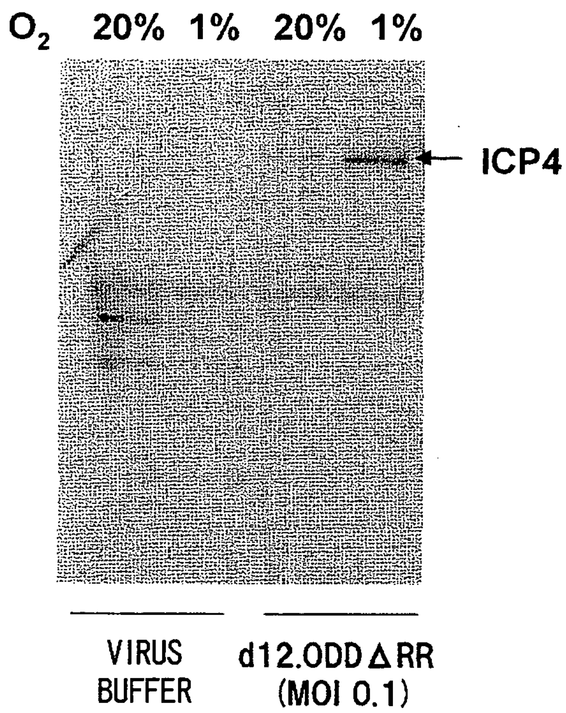
O2 20% 1% 20% 1%
ICP4
VIRUS BUFFER
d12.ODDΔRR (MOI 0.1)

FIG. 6

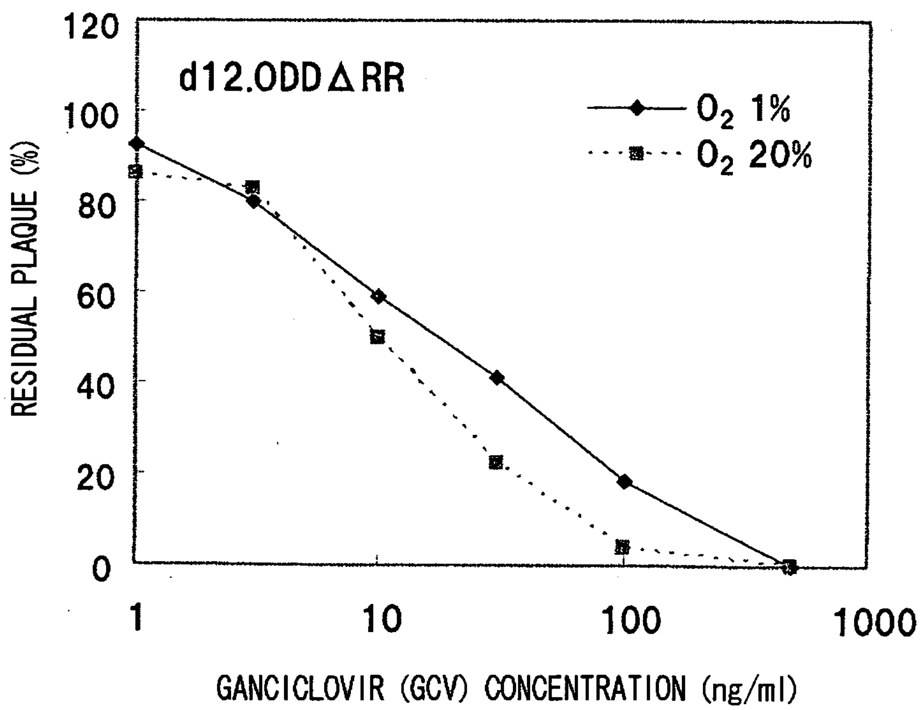
d12.ODDΔRR
O2 1%
O2 20%
RESIDUAL PLAQUE (%)
0
20
40
60
80
100
120
1
10
100
1000
GANCICLOVIR (GCV) CONCENTRATION (ng/ml)

FIG. 7

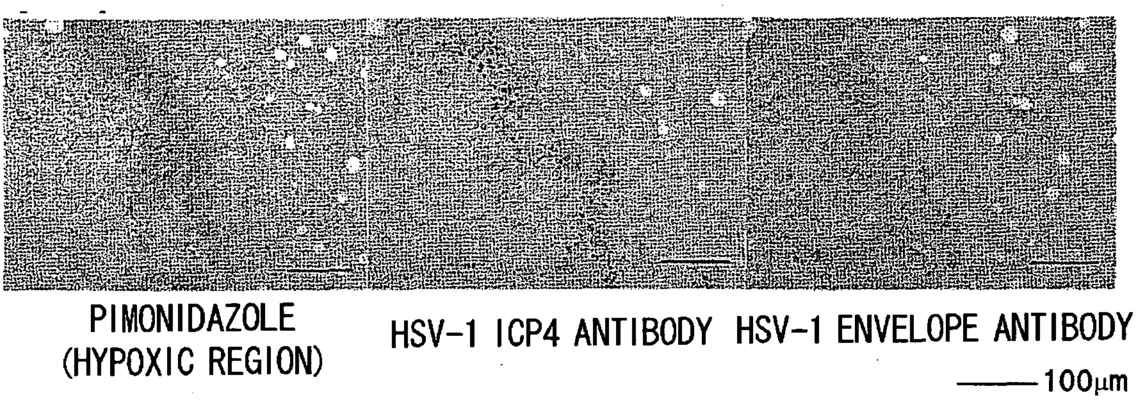
PIMONIDAZOLE
(HYPOXIC REGION)
HSV-1 ICP4 ANTIBODY
HSV-1 ENVELOPE ANTIBODY
100μm

FIG. 8

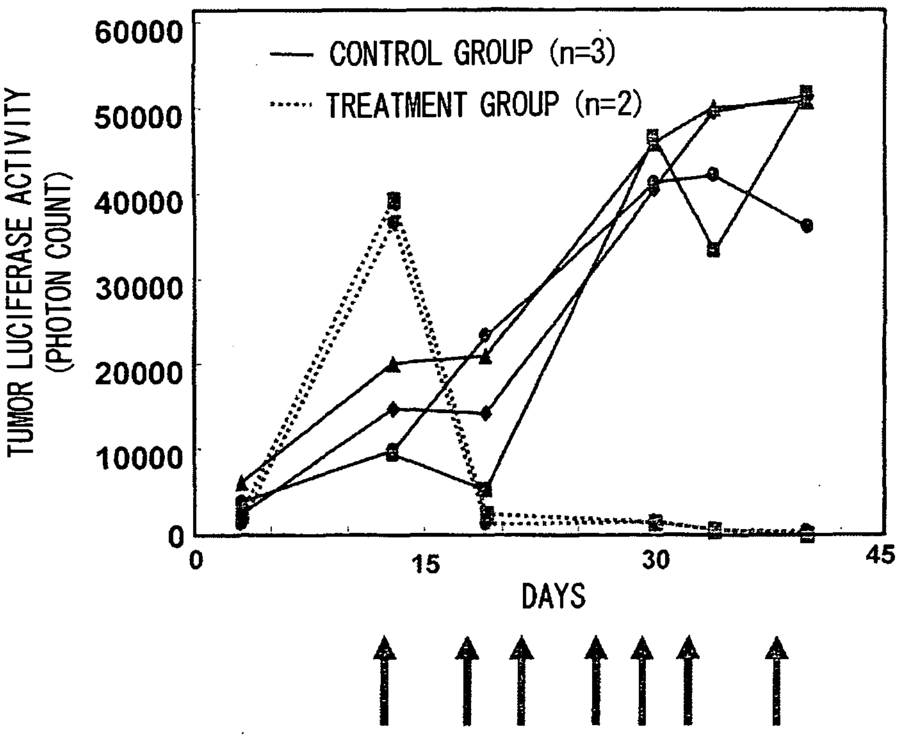
CONTROL GROUP (n=3)
TREATMENT GROUP (n=2)
TUMOR LUCIFERASE ACTIVITY
(PHOTON COUNT)
60000
50000
40000
30000
20000
10000
0
0
15
30
45
DAYS

FIG. 9

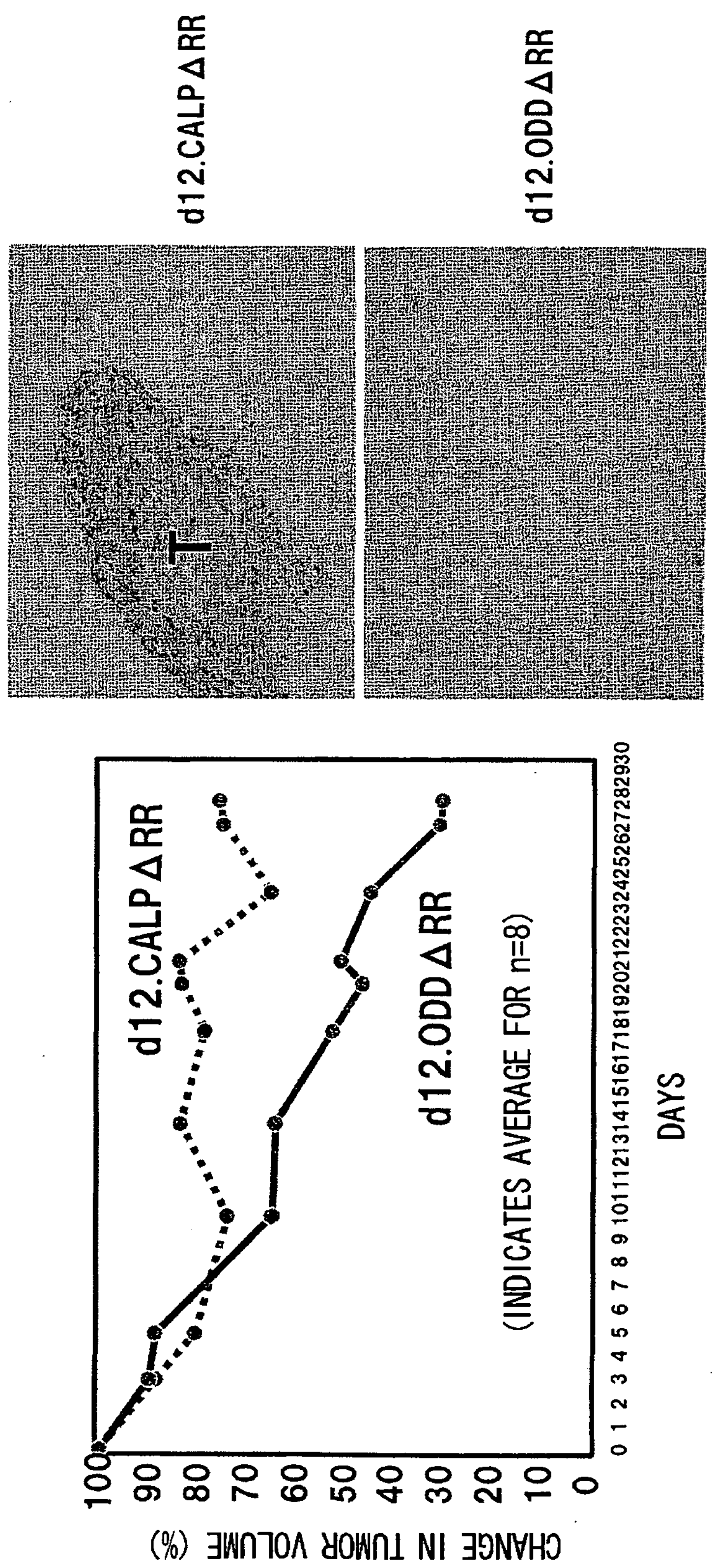
d12.CALPΔRR
d12.ODDΔRR
T
CHANGE IN TUMOR VOLUME (%)
100
90
80
70
60
50
40
30
20
10
0
0 1 2 3 4 5 6 7 8 9 10 11 12 13 14 15 16 17 18 19 20 21 22 23 24 25 26 27 28 29 30
DAYS
d12.CALPΔRR
d12.ODDΔRR
(INDICATES AVERAGE FOR n=8)

FIG. 10

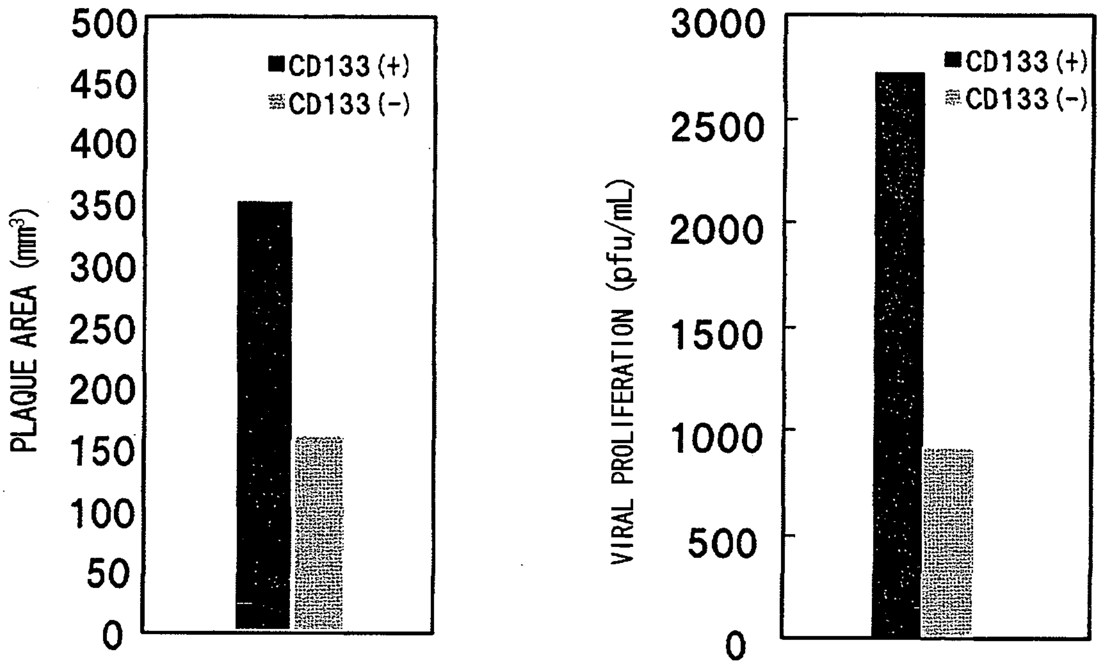
500
450
400
350
300
250
200
150
100
50
0
PLAQUE AREA (mm3)
CD133 (+)
CD133 (-)
3000
2500
2000
1500
1000
500
0
VIRAL PROLIFERATION (pfu/mL)
CD133 (+)
CD133 (-)

**INTERNATIONAL SEARCH REPORT**

International application No. PCT/JP2009/050299

A. CLASSIFICATION OF SUBJECT MATTER
*C12N15/09*(2006.01)i, *A61K35/76*(2006.01)i, *A61P9/10*(2006.01)i, *A61P11/00*(2006.01)i, *A61P25/00*(2006.01)i, *A61P35/00*(2006.01)i, *C07K14/03*(2006.01)i, *C07K14/47*(2006.01)i, *C07K19/00*(2006.01)i, *C12N7/00*(2006.01)i
According to International Patent Classification (IPC) or to both national classification and IPC

B. FIELDS SEARCHED
Minimum documentation searched (classification system followed by classification symbols)
C12N15/09, A61K35/76, A61P9/10, A61P11/00, A61P25/00, A61P35/00, C07K14/03, C07K14/47, C07K19/00, C12N7/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
BIOSIS/MEDLINE/WPIDS(STN), CAplus(STN), JSTPlus(JDreamII), GenBank/EMBL/DDBJ/GeneSeq, UniProt/GeneSeq, PubMed

C. DOCUMENTS CONSIDERED TO BE RELEVANT

| Category* | Citation of document, with indication, where appropriate, of the relevant passages | Relevant to claim No. |
|---|---|---|
| Y/A | WO 2003/057888 A1 (JAPAN SCIENCE AND TECHNOLOGY CORP.), 17 July, 2003 (17.07.03), Full text & JP 2003-250579 A & US 2005/0032214 A1 & EP 1469077 A1 | 1-6,8-16/7 |
| Y/A | HARADA, H., et al., Antitumor effect of TAT-oxygen-dependent degradation-caspase-3 fusion protein specifically stabilized and activated in hypoxic tumor cells., Cancer Research, 2002, Vol.62, p.2013-2018, full text | 1-6,8-16/7 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 February, 2009 (26.02.09) | 10 March, 2009 (10.03.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

INTERNATIONAL SEARCH REPORT

International application No.
PCT/JP2009/050299

C (Continuation). DOCUMENTS CONSIDERED TO BE RELEVANT

| Category* | Citation of document, with indication, where appropriate, of the relevant passages | Relevant to claim No. |
|---|---|---|
| Y/A | INOUE, M., et al., Targeting hypoxic cancer cells with a protein prodrug is effective in experimental malignant ascites., International Journal of Oncology, 2004, Vol.25, p.713-720, full text | 1-6,8-16/7 |
| Y/A | KOSHIKAWA, N., et al., Hypoxia-regulated expression of attenuated diphtheria toxin A fused with hypoxia-inducible factor-1alpha oxygen-dependent degradation domain preferentially induces apoptosis of hypoxic cells in solid tumor., Cancer Research, 2005, Vol.65, No.24, p.11622-30, full text | 1-6,8-16/7 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008007179 A
- JP 2006205006 A
- US 4937190 A


### Non-patent literature cited in the description


- **Sipkins D.A. et al.** *Nature,* 2005, vol. 435, 969-973
- **Ailles L.E. ; Weissman I.L.** *Curr. Opin. Biotech.,* 2007, vol. 18, 460-466
- **De Toni F. et al.** *Oncogene,* 2006, vol. 25, 3113-3122
- **Harada H. et al.** *Cancer Res.,* 2002, vol. 62, 2013-2018
- **Koshikawa N. ; Takenaga K.** *Cancer Res.,* 2005, vol. 65, 11622-11630
- **Cuevas Y. et al.** *Cancer Res.,* 2003, vol. 61, 6877-6884
- *J. Virol.,* 1985, vol. 56, 558-570
- *J. Virol.,* 1998, vol. 62, 196-205